# EUROPEAN PATENT APPLICATION

(11) **EP 4 144 327 A1**
(43) Date of publication of application: **08.03.2023**
(21) Application number: 22185245.2
(22) Date of filing: 15.07.2022
(51) Int. Cl.: A61F 2/30, A61F 2/42

(54) **SURGICAL IMPLANTS HAVING DELIVERY PORTS AND METHODS OF USING THE SAME**

(30) Priority: 07.09.2021 US 202163241223 P; 13.07.2022 US 202217812195
(71) Applicant: Wright Medical Technology, Inc., Memphis, TN 38117 (US)
(72) Inventor: McGinley, Shawn E., Arlington, 38002 (US); Tuttle, David R., Memphis, 38104 (US)
(74) Representative: Zimmermann & Partner Patentanwälte mbB

(57) **Abstract**

A device including a delivery port and a method of delivering a material via a delivery port is disclosed. The device includes a body (102) extending between a proximal surface (106) and a distal surface (104). The body is sized and configured to be coupled to an anatomical structure at an implantation site. A first inlet port (110) is formed in the distal surface of the body and is sized and configured to receive a first material. A first outlet port (136) is formed in the proximal surface of the body. The first outlet port is coupled to the first inlet port by a first fluid path (140, 150) defined by the body and the first outlet port is sized and configured to provide the first material to a first predetermined location when the body is coupled to the anatomical structure.

## Description

### Cross-Reference to Related Application

This application claims priority under 35 U.S.C. § 119(e) to United States Provisional Application No. 63/241,223, filed on September 7, 2021, the entire contents of which are incorporated herein by reference.

### Background of the Invention

During implant surgery, it may be necessary to provide drugs, biologics, bone cement, and/or other materials ("delivered material") on a bone interface side of an implant and/or between an implant and a bone. The delivered material may be provided to increase bone growth onto and/or into the implant, fill voids in a bone, and/or for any other suitable purpose.

Current materials typically include liquid or gel-like delivered materials. During surgery, it may be difficult to maintain the delivered materials in a fixed position while inserting an implant. For example, in some instances, the delivered material may be squeezed out or dislodged due to implant seating.

### Summary of the Invention

In various embodiments, a surgical implant includes a body extending between a proximal surface and a distal surface. The body is sized and configured to be coupled to an anatomical structure at an implantation site. A first inlet port is formed in a distal surface of the body. The inlet port is sized and configured to receive a first material. A first outlet port is formed in the proximal surface of the body. The first outlet port is coupled to the first inlet port by a first fluid path defined by the body. The first outlet port is sized and configured to provide the first material to a first predetermined location when the body is coupled to the anatomical structure.

In various embodiments, a surgical implant includes a body extending between a proximal surface and a distal surface. The body is sized and configured to be coupled to an anatomical structure at an implantation site, and wherein the proximal surface comprises a porous portion. A first inlet port is formed in a distal surface of the body. The inlet port is sized and configured to receive a first material. A first outlet port is formed in the proximal surface of the body. The first outlet port is coupled to the first inlet port by a first fluid path defined by the body. The first outlet port is sized and configured to provide the first material to the porous portion of the proximal surface.

In various embodiments, a method is provided for delivering a material within an implantation site in a patient. The method includes the step of positioning an implant adjacent to and in contact with at least one anatomical structure. The implant includes a body extending between a proximal surface and a distal surface sized and configured to be coupled to the anatomical structure at a predetermined location, a first inlet port formed in a distal surface of the body is sized and configured to receive a first material, and a first outlet port formed in the proximal surface of the body is coupled to the first inlet port by a first fluid path defined by the body. A first material is provided to the inlet port and the first material is delivered to the predetermined location by the first material passing through the first fluid path to the first outlet port.

Further disclosed herein is a device including a delivery port and a method of delivering a material via a delivery port is disclosed, wherein the device includes a body extending between a proximal surface and a distal surface, wherein the body is sized and configured to be coupled to an anatomical structure at an implantation site, wherein a first inlet port is formed in a distal surface of the body and is sized and configured to receive a first material, wherein a first outlet port is formed in the proximal surface of the body, wherein the first outlet port is coupled to the first inlet port by a first fluid path defined by the body and the first outlet port is sized and configured to provide the first material to a first predetermined location when the body is coupled to the anatomical structure.

### Brief Description of the Figures

The features and advantages of the invention will be more fully disclosed in, or rendered obvious by the following detailed description of preferred embodiments, which are to be considered together with the accompanying drawings wherein like numbers refer to like parts and further wherein:
FIG. 1 is a top perspective view of an implant including a plurality of input ports formed in a distal surface, in accordance with some embodiments.
FIG. 2 is a bottom perspective view of the implant of FIG. 1, showing a plurality of exit ports formed in a proximal surface of the implant of FIG. 1, in accordance with some embodiments.
FIG. 3 is a top plan view of the implant of FIG. 1, in accordance with some embodiments.
FIG. 4 is a cross-sectional view of the implant of FIG. 3 taken along line A-A, in accordance with some embodiments.
FIG. 5 is a cross-sectional view of the implant of FIG. 3 taken along line B-B, in accordance with some embodiments.
FIG. 6 is a top perspective view of a further embodiment of implant including a porous proximal body portion defining a porous proximal surface, in accordance with some embodiments.
FIG. 7 is a top perspective view of the implant of FIG. 6 with the porous proximal body portion shown in phantom, in accordance with some embodiments.
FIG. 8 a top plan view of the implant of FIG. 6, in accordance with some embodiments.
FIG. 9 is a cross-sectional view of the implant of FIGS. 6 and 8, taken along line C-C in FIG. 8, in accordance with some embodiments.
FIG. 10 is a cross-sectional view of the implant of FIG. 6 and 8 taken along line D-D in FIG. 8, in accordance with some embodiments.
FIG. 11 is a flowchart of a method of delivering a material at an implantation site, in accordance with some embodiments.

### Detailed Description of Preferred Embodiments

The description of the exemplary embodiments is intended to be read in connection with the accompanying drawings, which are to be considered part of the entire written description. In the description, relative terms such as "lower," "upper," "horizontal," "vertical," "proximal," "distal," "above," "below," "up," "down," "top" and "bottom," as well as derivatives thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

As used herein, the term "substantially" denotes elements having a recited relationship (e.g., parallel, perpendicular, aligned, etc.) within acceptable manufacturing tolerances. For example, as used herein, the term "substantially parallel" is used to denote elements that are parallel or that vary from a parallel arrangement within an acceptable margin of error, such as +/- 5°, although it will be recognized that greater and/or lesser deviations can exist based on manufacturing processes and/or other manufacturing requirements.

In various embodiments, a system and method for delivering a material to an implantation site are disclosed. The system includes an implant having at least one entry port formed in a distal surface of the implant. The entry port is sized and configured to receive a material therein. The material may include, but is not limited to, a liquid or gel-like drug, biologic, bone cement, and/or other therapeutic or restorative material. The entry port is in fluid-communication with one or more exit ports formed in a proximal surface and/or proximal structure of the implant. For example, in some embodiments, the entry port is coupled to one or more exit ports by an internal channel, although it will be appreciated that any suitable fluid coupling may be used. The one or more exit ports are sized and configured to deliver the liquid or gel-like delivered material to one or more predetermined positions after the implant is positioned in contact with a bone.

In various embodiments, the method includes a step of positioning an implant in contact with a bone at a predetermined implantation site. After positioning the implant, a liquid or gel-like material is provided to an entry port formed in a distal surface of the implant. The delivered material is guided from the entry port to one or more exit ports by one or more internal channels defined by the implant. The delivered material is provided to one or more predetermined locations with respect to the implantation site by the exit ports.

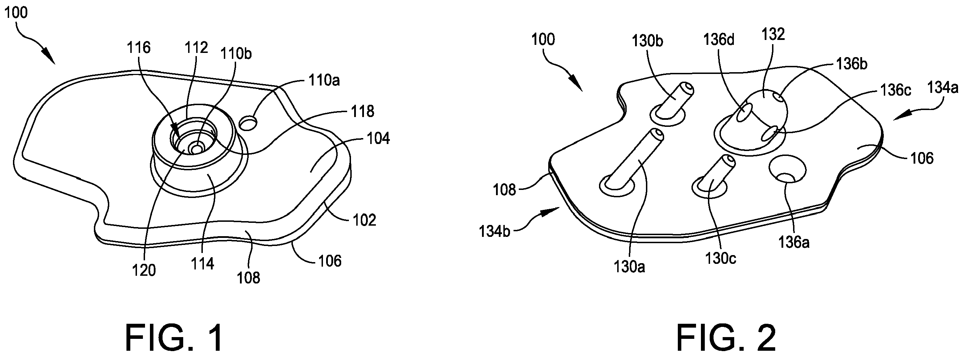

FIG. 1 illustrates a distal perspective view of an implant 100 including a plurality of inlet ports 110 formed in a distal surface 104, in accordance with some embodiments. FIG. 2 illustrates a proximal perspective view of the implant 100, in accordance with some embodiments. The implant 100 includes a surgical implant configured to be coupled to a bone. For example, in some embodiments, the implant 100 may include a bone implant configured to replace one or more joint surfaces and/or receive one or more structures configured to replace one or more joint surfaces. In other embodiments, the implant may include a structural implant and/or any other suitable type of bone implant.

The implant 100 includes a body 102 extending between a distal surface 104 and a proximal surface 106. A sidewall 108 defines a perimeter of the body 102. The sidewall 108 defines a predetermined shape of the implant configured to correspond to an implantation site. In some embodiments, the sidewall 108 is configured to match a contour of a patient bone, although it will be appreciated that the sidewall 108 may have any suitable shape, including a patient-specific and/or patient-universal shape.

In the illustrated embodiments, the distal surface 104 and the proximal surface 106 each define generally planar surfaces, although it will be appreciated that the surface topography may be configured to match the surface of a bone to which the implant is mounted. The distal surface 104 may include a protrusion 112, such as a peg, morse taper, and/or other protrusion extending distally from the distal surface 104 (FIG. 1). In the illustrated embodiment, the protrusion 112 includes a protrusion having a circumferential shape that defines a generally circular outer wall 114 and an inset portion 116 defined by a generally circular inner wall 118. The inset portion 116 is further defined by a proximal inset surface 120. Although a single protrusion 112 having a single inset portion 116 is illustrated, it will be appreciated that the implant 100 may include a plurality of protrusions 112 and/or each protrusion 112 may include a plurality of inset portions 116.

In some embodiments, the distal surface 104 defines one or more inlet ports 110a, 110b (collectively "inlet ports 110"). The inlet ports 110 may be defined through any portion of the distal surface 104 and/or any structure formed on the distal surface 104. For example, in some embodiments, a first inlet port 110a is formed in the distal surface 104 and a second inlet port 110b is formed in the inset surface 120. Although embodiments are discussed herein including a plurality of inlet ports 110, it will be appreciated that the first inlet port 110a and/or the second inlet port 110b may be omitted such that the implant 100 includes only a single inlet port 110. As discussed in greater detail below, the inlet ports 110 are in fluid communication with one or more outlet ports formed on a proximal surface of the implant 100.

In some embodiments, at least one of the inlet ports 110 is defined by an opening formed in the distal surface 104 of the implant 100 and extending at least partially into the body of the implant 100. In some embodiments, one or more of the inlet ports 110 may taper from a first diameter at the distal surface 104 to a second diameter at a predetermined distance into the body 102. In some embodiments, one or more of the inlet ports 110 defines a constant diameter.

Referring to FIG. 2, in some embodiments a plurality of pegs 130a-130c (collectively "pegs 130") extend proximally from the proximal surface 106. Each of the pegs 130 extends at a predetermined angle and a predetermined length with respect to the proximal surface 106. For example, in the illustrated embodiments, the first peg 130a extends at a first angle with respect to the proximal surface 106 and has a first longitudinal distance, a second peg 130b extends at a second angle with respect to the proximal surface 106 and has a second longitudinal distance, and a third peg 130c extends at a third angle with respect to the proximal surface 106 and has a third longitudinal distance. The first angle, the second angle, and/or the third angle may be equal. Similarly, the first longitudinal distance, the second longitudinal distance, and/or the third longitudinal distance may be equal.

Each of the pegs 130 are sized and configured to be received within a peg hole formed in a bone prior to positioning of the implant 100 adjacent to and in contact with the bone. For example, in embodiments including an implant 100 configured to be coupled to a joint surface for joint replacement, one or more peg holes may be formed in a resected bone to maintain the implant 100 in a fixed position. Although pegs 130 are discussed herein, it will be appreciated that the proximal surface 106 may include any suitable surface features, such as pegs, contours, surface treatments, etc. configured to maintain the implant 100 in a fixed position with respect to a bone.

Referring to FIGS. 2-4, in some embodiments the implant 100 includes a stem 132 projecting from the proximal surface 106. The stem 132 may project outwardly at an angle with respect to the proximal surface 106. The angle of the stem 132 may be equal to one or more of the angles defined between the pegs 130 and the proximal surface 106 and/or may be different than the angles defined between the pegs 130 and the proximal surface 106. In the illustrated embodiment, each of the pegs 130 and the stem 132 project outwardly toward a first end 134a of the implant 100, although it will be appreciated that one or more of the pegs 130 and/or the stem 132 may project in a direction toward a second end 134b, perpendicular to the proximal surface 106, and/or in any other suitable direction.

In some embodiments, the stem 132 is configured to be received within a stem hole formed in a bone prior to positioning of the implant 100 adjacent to and in contact with the bone. In some embodiments, the stem 132 is configured to position and/or orient the implant 100 with respect to the bone surface and/or one or more other features, such as, for example, alignment of a joint surface. Although multiple embodiments are discussed, including a stem 132, it will be appreciated that the stem 132 may be omitted.

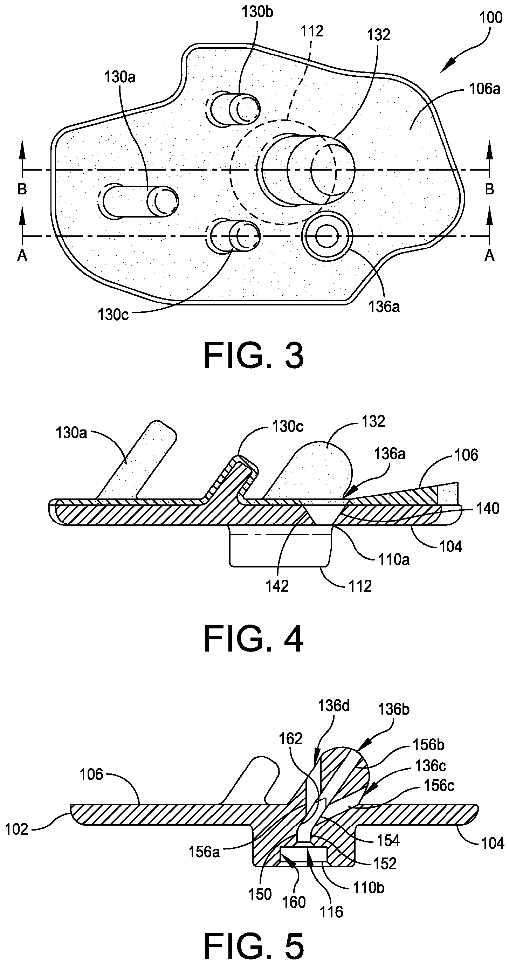

Referring to FIGS. 3-5, in some embodiments the proximal surface 106 defines one or more outlet ports 136a-136d (collectively "outlet ports 136"). The outlet ports 136 may be formed directly in the proximal surface, for example outlet port 136a, and/or may be formed in a structure coupled to and/or extending from the proximal surface 106, for example outlet ports 136b-136d. As discussed in further detail below, the outlet ports 136 are in fluid communication with one or more of the inlet ports 110 formed on the distal surface 104. The outlet ports 136 are sized and configured to deliver a predetermined quantity of a delivered material inserted into the inlet ports 110 to predetermined positions with respect to the implant 100. When the implant 100 is positioned adjacent to and in contact with a bone, the outlet ports 136 are configured to provide the delivered material to predetermined locations relative to the implantation site and the implant 100.

For example, in some embodiments, one or more of the outlet ports 136 are positioned to deliver a fixation material, such as a bone cement, to one or more predetermined locations at an implantation site after positioning of the implant 100 adjacent to and in contact with the implantation site. As another example, in some embodiments, one or more of the outlet ports 136 may be configured to provide a biological treatment material, such as drugs, biologics, bone-growth material, etc., to one or more predetermined locations at an implantation site.

Referring again to FIGS. 1 and 2, in some embodiments a first inlet port 110a may be in fluid communication with a first set of the outlet ports 136, such as outlet port 136a, and a second inlet port 110b may be in fluid communication with a second set of the outlet ports 136, such as outlet ports 136b-136d. A first material may be provided to the first inlet port 110a for delivery to a location adjacent to the first set of outlet ports and a second material may be provided to the second inlet port 110b for delivery to a location adjacent to the second set of outlet ports. The first material and the second material may be the same material, a different material, and/or a combination of similar and different materials.

Referring to FIGS. 3-4, in some embodiments a first inlet port 110a formed in a distal surface 104 of the implant 100 is in fluid communication with a first outlet port 136a formed in the proximal surface 106. The first inlet port 110a is coupled to the first outlet port 136a via a continuously tapered fluid path 140 (FIG. 4). The continuously tapered fluid path 140 extends directly from the inlet port 110a to the outlet port 136a (FIG. 3) such that the inlet port 110a and the outlet port 136a define a single channel extending through the body 102 of the implant 100. In some embodiments, the sidewall 142 of the continuously tapered fluid path 140 is defined by the material of the body 102 of the implant 100. In some embodiments, an inner liner or coating may be applied to the sidewall 142 to create an additional barrier between the delivered material and the material of the body 102.

Referring to FIGS. 3 and 5, in some embodiments a second inlet port 110b is coupled to a plurality of outlet ports 136b-136d formed in a stem 132 of the implant 100. The second inlet port 110b is coupled to the plurality of outlet ports 136b-136d via a branching fluid path 150. The branching fluid path 150 includes a first channel 152 extending from the second inlet port 110b proximally into the body 102 of the implant 100 to a second channel 154. The second channel 154 extends from the first channel 152 at a predetermined angle. In some embodiments, the first channel 152 is centered with respect to the insert portion 116, although it will be appreciated that the first channel 152 may be offset from the center of the inset portion 116.

In some embodiments, a plurality of exit channels 156a-156c (collectively "exit channels 156") extend from the first channel 152 and/or the second channel 154 to one of the plurality of outlet ports 136b-136d. In some embodiments, each of the exit channels 156 extends from a proximal end of the first channel 154, although it will be appreciated that the exit channels 156 may be coupled to the first channel 152 and/or the second channel 154 at any suitable location. As shown in FIGS. 1 and 5, in some embodiments, the inset portion 116 includes a first circumferential cross-section having a first diameter 160 and the first channel 152 includes a second circumferential cross-section having a second diameter 162, that is less than the first diameter. Although embodiments are shown with circumferential cross-sections, it will be appreciated that the inset portion 116, the first channel 152, the second channel 154, and/or the exit channels 156 may include any suitable cross-sectional shape.

In some embodiments, the implant 100 is formed by an additive manufacturing process, such as, for example, sintering processes, melting processes, and/or stereolithography processes. Such additive manufacturing processes may include, but are not limited to, binder jetting, directed energy deposition, material extrusion, powder bed fusion, sheet lamination, vat polymerization, Directed Energy Deposition-Arc (DED-arc) (*e.g.,* wire arc additive manufacturing), and/or any other suitable additive manufacturing process. In some embodiments, the implant 100 may be formed as a single, monolithic implant. In some embodiments, portions of the implant 100 may be formed separately by additive manufacturing and/or traditional manufacturing and coupled together to form implant 100.

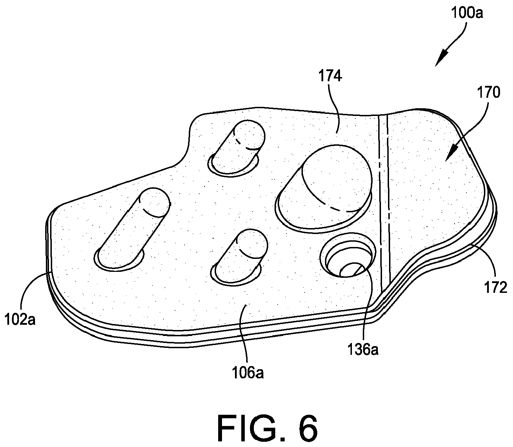

Referring to FIG. 6, an implant 100a including a porous proximal body portion 170 defining a porous proximal surface 106a, in accordance with some embodiments. The implant 100a is similar to the implant 100 discussed above in conjunction with FIGS. 1-5, and similar description is not repeated herein. The porous proximal body portion 170 is configured to provide fluid communication from a distal side 172 to a proximal side 174. In some embodiments, the porous proximal body portion 170 defines the entire proximal portion and proximal surface 106a of the body 102a, although it will be appreciated that the porous proximal body portion 170 may extend over only a portion of the proximal side of the implant 100a.

The porous proximal body portion 170 and the porous proximal surface 106a are configured to allow fluid transfer from one or more exit ports 136 through the porous proximal surface 106a to one or more locations at an implant site. In some embodiments, the porous proximal body portion 170 is configured to distribute the delivered material over a larger surface area of the proximal surface 106a as compared to delivery exclusively through the exit ports 136. For example, in some embodiments, the porous proximal body portion 170 may allow the delivered material to substantially distribute over the entirety of the proximal surface 106a and/or some predetermined portion of the proximal surface 106a, allowing distribution of the delivered material over the entirety and/or a predetermined portion of an implantation site.

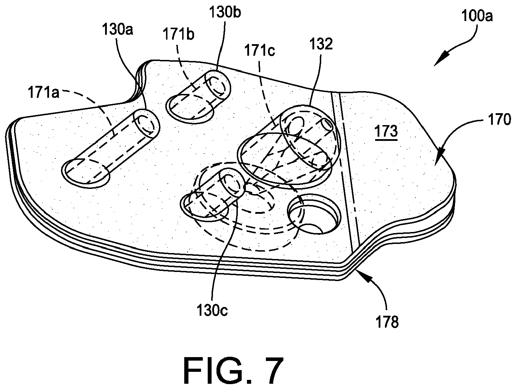

Referring to FIG. 7, in some embodiments, the porous proximal body portion 170 defines one or more structures 171a, 171c and/or cavities 171b sized and shaped so as to accept entry of corresponding elements, e.g., one or more pegs 130 and/or stem 132, of the non-porous distal body portion 178. In some embodiments, the non-porous distal body portion 178 may define a proximal surface configured to be interfaced with a distal surface of and/or may be formed integrally with the porous proximal body portion 170. In some embodiments, the porous proximal body portion 170 includes a contoured surface 173 that extends to the one or more pegs 130 and/or stem 132 of the non-porous distal body portion 178. For example, in the illustrated embodiment, the non-porous distal body portion 178 includes a plurality of pegs 130 and a stem 132 and the porous proximal body portion 170 includes a continuous porous surface extending over the plurality of pegs 130 and the stem 132.

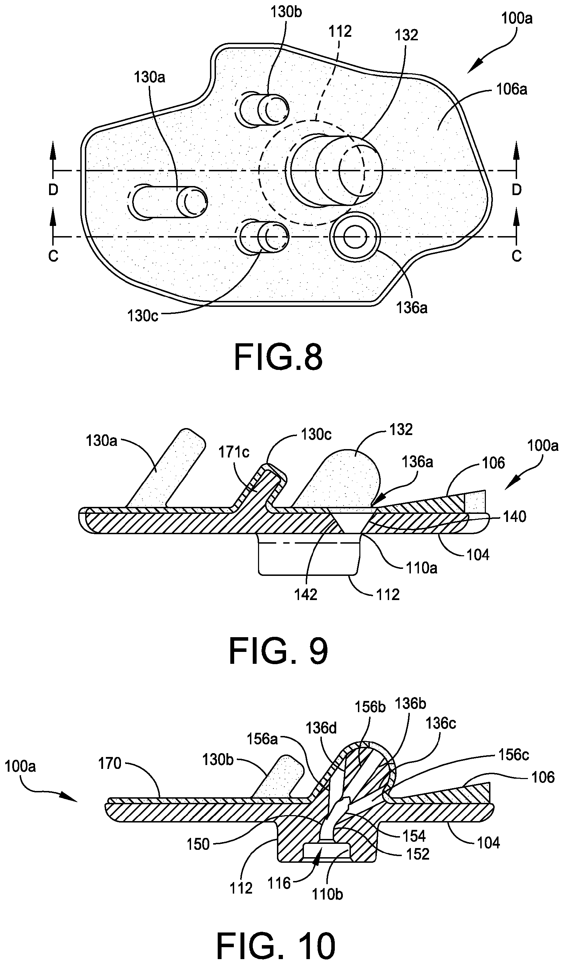

Referring to FIGS. 8-9 in some embodiments, a first inlet port 110a formed in a distal surface 104 of the implant 100a is in fluid communication with a first outlet port 136a formed in the proximal porous surface 106a. The first inlet port 110a is coupled to the first outlet port 136a via a continuously tapered fluid path 140a defined through the non-porous distal body portion 104 and the porous proximal body portion 106. In some embodiments, the continuously tapered fluid path 140a allows a material delivered to the first inlet port 110a may be substantially delivered through the first outlet port 136a to a predetermined location and the porous proximal body portion 170 allows some of the delivered material to diffuse around the first outlet port 136a. It will be appreciated that the amount of diffusion may be controlled by adjusting the porosity of the porous proximal body portion 170.

Referring to FIGS. 8 and 10, in some embodiments, a second inlet port 110b is coupled to a plurality of outlet ports 136b-136d formed in a stem 132 of the implant 100. Each of the plurality of outlet ports 136b-136d is covered by a portion of the porous proximal body portion 170. A material delivered to the second entry port 110b is transported through the branching fluid path 150 to each of the plurality of outlet ports 136b-136c. The delivered material exits the outlet ports 136b-136d and is diffused through the porous proximal body portion 170 and delivered over at least a portion of the porous proximal surface 106a. It will be appreciated that the lateral distance covered by the delivered material (*i.e*., the portion of the porous proximal surface 106a through which the delivered material exits) is determined by the porosity of the porous proximal body portion 170.

For example, in some embodiments, the porous proximal body portion 170 has a porosity configured to provide the delivered material over substantially most of the proximal planar surface 106a co-located with the stem 132. As another example, in some embodiments, the porous proximal body portion 170 has a porosity configured to provide the delivered material to predetermined portions of the porous proximal body portion 170 co-located with the stem 132. Although various embodiments are discussed herein, it will be appreciated that any suitable porosity and/or porosity gradient may be selected to provide a desired diffusion of a delivered material.

As discussed above with respect to implant 100, the implant 100a may be formed using an additive manufacturing process, such as, for example, sintering processes, melting processes, and/or stereolithography processes. Such additive manufacturing processes may include, but are not limited to, binder jetting, directed energy deposition, material extrusion, powder bed fusion, sheet lamination, vat polymerization, Directed Energy Deposition-Arc (DED-arc) (*e.g.,* wire arc additive manufacturing), and/or any other suitable additive manufacturing process. In some embodiments, the implant 100a may be formed as a single, monolithic implant having the porous proximal body portion 170 formed integrally with the non-porous distal body portion 172 during the additive manufacturing process. In some embodiments, portions of the implant 100, such as the porous proximal body portion 170 and the non-porous distal body portion 172, may be formed separately by additive manufacturing and/or traditional manufacturing and coupled together to form implant 100.

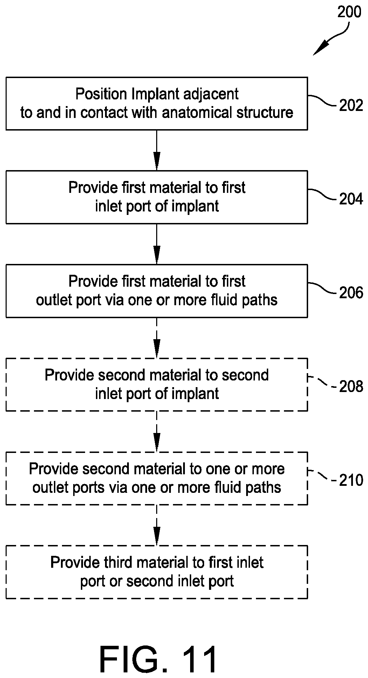

FIG. 11 is a flowchart illustrating a method of delivering a material at an implantation site, in accordance with some embodiments. At step 202, an implant, such as implant 100 or 100a discussed above, is positioned adjacent to and in contact with an anatomical structure, such as a bone, at a prepared implantation site. The implantation site may be prepared prior to positioning of the implant using one or more guide systems, cutting instruments, and/or other preparation systems and devices.

At step 204, after positioning the implant, a first material is provided to a first inlet port 110a of the implant. The first material may include any suitable biocompatible material, including, but not limited to, a liquid or gel-like material. Examples of suitable materials include, but are not limited to, drugs, biologics, bone cement, and/or other suitable materials.

At step 206, the first material is provided to one or more predetermined locations with respect to the implantation site via one or more outlet ports, such as a first outlet port 132a. The first material may be provided from the first inlet port 110a to the first outlet port 132a via one or more fluid paths, such as a continuously tapered fluid path 140. In some embodiments, the first outlet port 132a opens directly to a predetermined location with respect to the implantation site. In some embodiments, the first outlet port 132a may interact with one or more additional elements, such as a porous proximal body portion 170.

At optional step 208, a second material is provided to a second inlet port 110a of the implant. The second material may include any suitable biocompatible material, including, but not limited to, a liquid or gel-like material. Examples of suitable materials include, but are not limited to, drugs, biologics, bone cement, and/or other suitable materials. The second material may be the same material as the first material or a different material.

At optional step 210, the second material is provided to one or more predetermined locations with respect to the implantation site via one or more outlet ports, such as a plurality of outlet ports 132b-132d. The second material may be provided from the second inlet port 110a to the plurality of outlet ports 132b-132d via one or more fluid paths, such as a branching fluid path 150. In some embodiments, the plurality of outlet ports 132b-132d each open directly to a predetermined location with respect to the implantation site. In some embodiments, the plurality of outlet ports 132b-132d interact with one or more additional elements, such as a porous proximal body portion 170.

At optional step 212, a third material is provided to the first inlet port 110a and/or the second inlet port 110b. The third material may include any suitable biocompatible material, including, but not limited to, a liquid or gel-like material. Examples of suitable materials include, but are not limited to, drugs, biologics, bone cement, and/or other suitable materials. The third material may be the same material as the first material, the same as the second material, or a different material.

Although the subject matter has been described in terms of exemplary embodiments, it is not limited thereto. Rather, the appended claims should be construed broadly, to include other variants and embodiments, which may be made by those skilled in the art.

Further disclosed herein is the subject-matter of the following clauses:
1. A device, comprising:
   a body extending between a proximal surface and a distal surface, wherein the body is sized and configured to be coupled to an anatomical structure at an implantation site;
   a first inlet port formed in a distal surface of the body, wherein the inlet port is sized and configured to receive a first material;
   a first outlet port formed in the proximal surface of the body, wherein the first outlet port is coupled to the first inlet port by a first fluid path defined by the body, and wherein the first outlet port is sized and configured to provide the first material to a first predetermined location when the body is coupled to the anatomical structure.
2. The device of clause 1, wherein the body comprises a non-porous distal portion and a porous proximal portion, and wherein the distal surface is defined by the non-porous distal portion and the proximal surface is defined by the porous proximal portion.
3. The device of clause 2, wherein the porous proximal portion extends at least partially over the first outlet port, and wherein the first material is provided to the predetermined location through the porous proximal portion.
4. The device of clause 1 or of any of the preceding clauses, wherein the first fluid path comprises a continuously tapered fluid path.
5. The device of clause 1 or of any of the preceding clauses, comprising a second outlet port formed in the proximal surface of the body, wherein the second outlet port is coupled to the first inlet port by the first fluid path, wherein the second outlet port is sized and configured to provide the first material to a second predetermined location when the body is coupled to the anatomical structure.
6. The device of clause 5, wherein the first fluid path comprises a branching fluid path.
7. The device of clause 1 or of any of clauses 1-4, comprising:
   a second inlet port formed in a distal surface of the body, wherein the second inlet port is sized and configured to receive a second material; and
   a second outlet port formed in a proximal surface of the body, wherein the second outlet port is coupled to the second inlet port by a second fluid path defined by the body, and wherein the second outlet port is sized and configured to provide the second material to a second predetermined location when the body is coupled to the anatomical structure.
8. The device of clause 1 or of any of the preceding clauses, comprising a protrusion extending from the distal surface of the body, wherein the first inlet port is formed in the protrusion.
9. The device of clause 1 or of any of the preceding clauses, comprising a stem extending from the proximal surface of the body, wherein the first outlet port is formed through a surface of the stem.
10. The device of clause 1 or of any of the preceding clauses, comprising a porous proximal portion disposed over a portion of the proximal surface of the body, wherein the porous proximal portion is configured to distribute the material received through the first inlet port over a predetermined area.
11. The device of clause 1 or of any of the preceding clauses, wherein the body is formed by an additive manufacturing process.
12. A device, comprising:
   a body extending between a proximal surface and a distal surface, wherein the body is sized and configured to be coupled to an anatomical structure at an implantation site, and wherein the proximal surface comprises a porous portion;
   a first inlet port formed in a distal surface of the body, wherein the inlet port is sized and configured to receive a first material;
   a first outlet port formed in the proximal surface of the body, wherein the first outlet port is coupled to the first inlet port by a first fluid path defined by the body, and wherein the first outlet port is sized and configured to provide the first material to the porous portion of the proximal surface.
13. The device of clause 11 or 12, wherein the first fluid path comprises one of a continuously tapered fluid path or a branching fluid path
14. The device of clause 11 or 12 or of any of clauses 11-13, comprising a second outlet port formed in the proximal surface of the body, wherein the second outlet port is coupled to the first inlet port by the first fluid path, wherein the second outlet port is sized and configured to provide the first material to a second predetermined location when the body is coupled to the anatomical structure.
15. The device of clause 11 or 12 or of any of clauses 11-14, comprising:
   a second inlet port formed in a distal surface of the body, wherein the second inlet port is sized and configured to receive a second material; and
   a second outlet port formed in a proximal surface of the body, wherein the second outlet port is coupled to the second inlet port by a second fluid path defined by the body, and wherein the second outlet port is sized and configured to provide the second material to a second predetermined location when the body is coupled to the anatomical structure.
16. The device of clause 11 or 12 or of any of clauses 11-15, comprising a protrusion extending from the distal surface of the body, wherein the first inlet port is formed in the protrusion.
17. The device of clause 11 or 12 or of any of clauses 11-16, comprising a stem extending from the proximal surface of the body, wherein the first outlet port is formed through a surface of the stem.
18. The device of clause 11 or 12 or of any of clauses 11-17, wherein the body is formed by an additive manufacturing process.
19. A method, comprising:
   positioning an implant adjacent to and in contact with at least one anatomical structure, wherein the implant comprises a body extending between a proximal surface and a distal surface sized and configured to be coupled to the anatomical structure at a predetermined location, a first inlet port formed in a distal surface of the body sized and configured to receive a first material, and a first outlet port formed in the proximal surface of the body coupled to the first inlet port by a first fluid path defined by the body;
   providing a first material to the inlet port; and
   delivering the first material to the predetermined location by the first material passing through the first fluid path to the first outlet port.
20. The method of clause 19, comprising, prior to delivering the first material to the predetermined location, diffusing the first material within a permeable layer of the implant, wherein the permeable layer is positioned adjacent to and in contact with the anatomical structure at the predetermined location.

## Claims

1. A device, comprising:
a body extending between a proximal surface and a distal surface, wherein the body is sized and configured to be coupled to an anatomical structure at an implantation site;
a first inlet port formed in a distal surface of the body, wherein the inlet port is sized and configured to receive a first material;
a first outlet port formed in the proximal surface of the body, wherein the first outlet port is coupled to the first inlet port by a first fluid path defined by the body, and wherein the first outlet port is sized and configured to provide the first material to a first predetermined location when the body is coupled to the anatomical structure.

2. The device of claim 1, wherein the body comprises a non-porous distal portion and a porous proximal portion, and wherein the distal surface is defined by the non-porous distal portion and the proximal surface is defined by the porous proximal portion.

3. The device of claim 2, wherein the porous proximal portion extends at least partially over the first outlet port, and wherein the first material is provided to the predetermined location through the porous proximal portion.

4. The device of any of the preceding claims, wherein the first fluid path comprises a continuously tapered fluid path.

5. The device of any of the preceding claims, comprising a second outlet port formed in the proximal surface of the body, wherein the second outlet port is coupled to the first inlet port by the first fluid path, wherein the second outlet port is sized and configured to provide the first material to a second predetermined location when the body is coupled to the anatomical structure.

6. The device of claim 5, wherein the first fluid path comprises a branching fluid path.

7. The device of any of claims 1-4, comprising:
a second inlet port formed in a distal surface of the body, wherein the second inlet port is sized and configured to receive a second material; and
a second outlet port formed in a proximal surface of the body, wherein the second outlet port is coupled to the second inlet port by a second fluid path defined by the body, and wherein the second outlet port is sized and configured to provide the second material to a second predetermined location when the body is coupled to the anatomical structure.

8. The device of any of the preceding claims, comprising a protrusion extending from the distal surface of the body, wherein the first inlet port is formed in the protrusion, and/or comprising a stem extending from the proximal surface of the body, wherein the first outlet port is formed through a surface of the stem.

9. The device of any of the preceding claims, comprising a porous proximal portion disposed over a portion of the proximal surface of the body, wherein the porous proximal portion is configured to distribute the material received through the first inlet port over a predetermined area.

10. The device of any of the preceding claims, wherein the body is formed by an additive manufacturing process.

11. A device, comprising:
a body extending between a proximal surface and a distal surface, wherein the body is sized and configured to be coupled to an anatomical structure at an implantation site, and wherein the proximal surface comprises a porous portion;
a first inlet port formed in a distal surface of the body, wherein the inlet port is sized and configured to receive a first material;
a first outlet port formed in the proximal surface of the body, wherein the first outlet port is coupled to the first inlet port by a first fluid path defined by the body, and wherein the first outlet port is sized and configured to provide the first material to the porous portion of the proximal surface.

12. The device of claim 11, wherein the first fluid path comprises one of a continuously tapered fluid path or a branching fluid path

13. The device of any of claims claim 11-12, comprising a second outlet port formed in the proximal surface of the body, wherein the second outlet port is coupled to the first inlet port by the first fluid path, wherein the second outlet port is sized and configured to provide the first material to a second predetermined location when the body is coupled to the anatomical structure.

14. The device of any of claims 11-13, comprising:
a second inlet port formed in a distal surface of the body, wherein the second inlet port is sized and configured to receive a second material; and
a second outlet port formed in a proximal surface of the body, wherein the second outlet port is coupled to the second inlet port by a second fluid path defined by the body, and wherein the second outlet port is sized and configured to provide the second material to a second predetermined location when the body is coupled to the anatomical structure.

15. The device of any of claims 11-14, comprising a protrusion extending from the distal surface of the body, wherein the first inlet port is formed in the protrusion, and/or comprising a stem extending from the proximal surface of the body, wherein the first outlet port is formed through a surface of the stem, and/or wherein the body is formed by an additive manufacturing process.
